(19) Europäisches Patentamt
European Patent Office
Office européen des brevets

(11) Veröffentlichungsnummer: **0 281 841**
**B1**

(12) # EUROPÄISCHE PATENTSCHRIFT

(45) Veröffentlichungstag der Patentschrift: **14.11.90**

(51) Int. Cl.⁵: **C07D 213/73**, C07D 213/74

(21) Anmeldenummer: **88102675.1**

(22) Anmeldetag: **24.02.88**

(54) **Verfahren zur Herstellung von 5-Amino-4,6-dihalogenpyridinen.**

(30) Priorität: **07.03.87 DE 3707361**
**13.03.87 DE 3708093**

(43) Veröffentlichungstag der Anmeldung:
**14.09.88 Patentblatt 88/37**

(45) Bekanntmachung des Hinweises auf die Patenterteilung:
**14.11.90 Patentblatt 90/46**

(84) Benannte Vertragsstaaten:
**BE CH DE ES FR GB IT LI NL SE**

(56) Entgegenhaltungen:
**EP-A- 0 244 728**
**DD-A- 154 537**
**US-A- 4 358 455**

(73) Patentinhaber: **BAYER AG,**
**D-5090 Leverkusen 1 Bayerwerk(DE)**

(72) Erfinder: **Hallenbach, Werner, Dr., Kleiststrasse 10,**
**D-4018 Langenfeld(DE)**
Erfinder: **Lindel, Hans, Dr., Carl-Duisberg-Strasse 321,**
**D-5090 Leverkusen(DE)**

**EP 0 281 841 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 5-Amino-4,6-dihalogen-pyridinen durch Halogenierung der entsprechenden 5-Aminopyridine mit Halogenwasserstoffsäure in Gegenwart von Oxidationsmitteln.

Die Halogenierung von in 2-Stellung substituierten 5-Nitropyridinen ist bekannt. Die Bromierung von 2-Amino-5-nitropyridin erfolgt in 3-Stellung; R.H. Brignell J. Chem. Soc. (B) (1970) 117-21. Ist die 3-Stellung substituiert z.B. bei 3-Amino-pyridin erfolgt die Halogenierung mit Halogenwasserstoffsäure in Gegenwart von Oxidationsmitteln in 2-Stellung (O.v. Schickh et. al. Ber. dt. Chem. Ges. 69, 2593 - 2605).

Über die selektive Halogenierung von 2-substituierten 5-Aminopyridinen in 4,6-Stellung war nichts bekannt.

Es wurde gefunden:

1. Verfahren zur Herstellung von 5-Amino-4,6-dihalogenpyridinderivaten der Formel

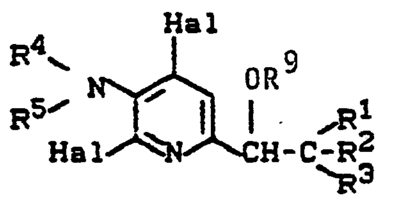

I

in welcher
$R^1$ für Wasserstoff, $-O-R^7$ oder $-NHR^8$ steht,
$R^2$ für Wasserstoff, Halogen steht
$R^3$ für Wasserstoff, oder gemeinsam mit $R^2$ für =O steht,
$R^4$ für Wasserstoff oder Alkyl steht,
$R^5$ für Wasserstoff, Alkyl, Halogenalkyl, Aryl steht,
$R^7$ für Alkyl,
$R^8$ für Alkyl, Cycloalkyl, Aralkyl, Aryl, Heterocyclyl steht, die gegebenenfalls substituiert sein können,
$R^9$ für Acyl oder Wasserstoff,
Hal für Halogen steht,
dadurch gekennzeichnet, daß man
Verbindungen der Formel II

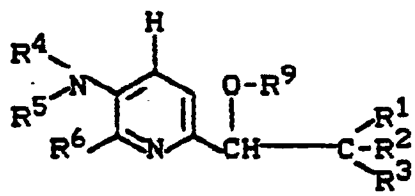

II

in welcher
$R^1$-$R^5$ die oben angegeben Bedeutung besitzen,
$R^6$ für Wasserstoff oder Halogen steht,
$R^9$ für Acyl oder Wasserstoff steht,
mit Halogenwasserstoffsäuren in Gegenwart von Oxidationsmitteln halogeniert.

2. 5-Aminopyridine der Formel III

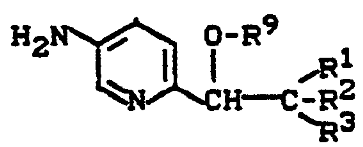

III

in welcher
$R^1$ für Wasserstoff, $-O-R^7$ oder $-NHR^8$ steht,
$R^2$ für Wasserstoff oder Halogen steht,

$R^3$ für Wasserstoff oder gemeinsam mit $R^2$ für =O steht,
$R^9$ für Acyl oder Wasserstoff steht,
$R^7$-$R^8$ die weiter oben genannten Bedeutungen besitzen,
sind neu.

3. Verfahren zur Herstellung von 5-Aminopyridinen der Formel III

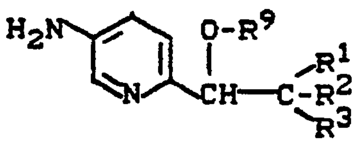

III

in welcher
$R^1$, $R^2$, $R^3$, $R^9$ die weiter oben genannten Bedeutungen besitzen,
dadurch gekennzeichnet, daß man 5-Nitropyridine der Formel IV

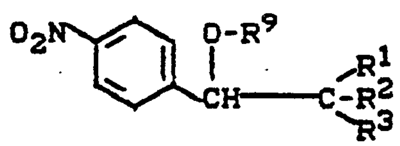

IV

in welcher
$R^1$, $R^2$, $R^3$, $R^9$ die weiter oben genannten Bedeutungen besitzen,
mit Wasserstoff katalytisch reduziert.

5-Amino-6-halogenpyridine der Formel I und ihre Herstellung sind auch Gegenstand einer nicht vorveröffentlichten Anmeldung derselben Anmelderin (EP-A O 244 728). Sie werden dort erhalten indem man Pyridinaldehyde mit Isonitrilen zu den entsprechenden α-Acetoxy-α-pyridylessigsäure-amiden umsetzt und diese anschließend gegebenenfalls hydrolysiert.

Die Monohalogenierung von 3-Aminopyridin war bekannt. Es war jedoch nicht bekannt, daß im Fall von 2-substituierten-5-Aminopyridinen eine Dihalogenierung selektiv in 4,6-Position erfolgt wenn man mit Halogenwasserstoffsäuren in Gegenwart von Oxidationsmitteln arbeitet. So ergab z.B. die Halogenierung mit Chlor oder Brom perhalogenierte Produkte. Auch bei der Halogenierung mit Halogenwasserstoffsäure in Gegenwart von Oxidationsmitteln wären Gemische von Halogenierungsprodukten mit unterschiedlichem Halogengehalt zu erwarten gewesen.

Bevorzugt werden nach dem erfindungsgemäßen Verfahren 5-Amino-4,6-dihalogenpyridinderivate der Formel I hergestellt
in welcher
$R^1$ für Wasserstoff, -O-$R^7$ oder -NH$R^8$ steht,
$R^2$ für Wasserstoff, Chlor oder Brom steht,
$R^3$ für Wasserstoff oder gemeinsam mit $R^2$ für doppelt gebundenen Sauerstoff (Carbonylsauerstoff) steht,
$R^4$ für Wasserstoff oder $C_{1-4}$-Alkyl steht,
$R^5$ für Wasserstoff, $C_{1-4}$-Alkyl, $C_{1-4}$-Alkylcarbonyl, Benzoyl, Benzylsulfonyl, Phenylsulfonyl das gegebenenfalls substituiert ist, $C_{1-4}$-Alkylsulfonyl steht. Als Substituenten kommen CN, Halogen, OH, $C_{1-4}$-Alkyl, $C_{1-4}$-Halogenalkyl, $C_{1-4}$-Alkoxy, $C_{1-4}$-Halogenalkoxy, $C_{1-4}$-Alkylthio, $C_{1-4}$-Halogenalkylthio infrage,
$R^7$ für $C_{1-6}$-Alkyl
$R^8$ für gegebenenfalls substituiertes $C_{1-6}$-Alkyl, $C_{3-6}$-Cycloalkyl, $C_{1-6}$-Alkylphenyl, Phenyl steht
$R^9$ für Alkylcarbonyl oder Wasserstoff steht.

Als Substituenten der gegebenenfalls substituierten Reste kommen bevorzugt infrage: Cyano, Halogen wie Fluor oder Chlor, Hydroxy, $C_{1-4}$-Alkyl, $C_{1-4}$-Halogenalkyl, Phenyl, $C_{1-4}$-Alkoxy, $C_{1-4}$-Halogenalkoxy, $C_{1-4}$-Alkyl thio, $C_{1-4}$-Halogenalkylthio, für den Fall, daß die Substituenten an einem Phenylrest sitzen, zusätzlich bevorzugt Methylendioxy, Ethylendioxy, halogensubstituiertes Methylendioxy, halogensubstituiertes Ethylendioxy, ferner Phenyl, Phenoxy, die ihrerseits einen oder mehrere der obengenannten Substituenten tragen können.

Hal für Chlor oder Brom steht.

Ganz besonders bevorzugt werden nach dem erfindungsgemäßen Verfahren 5-Amino-4,6-dihalogenpyridinderivate der Formel I hergestellt

in welcher
$R^1$ für Wasserstoff oder $-NHR^8$ steht,
$R^2$ für Wasserstoff oder Chlor steht,
$R^3$ für Wasserstoff oder gemeinsam mit $R^2$ für doppelt gebundenen Sauerstoff (Carbonylsauerstoff) steht,
$R^4$ für Wasserstoff steht,
$R^5$ für Wasserstoff steht,
$R^8$ für Wasserstoff, gegebenenfalls durch 1-5 Halogenatome oder $C_{3-6}$-Cycloalkyl substituiertes $C_{1-6}$-Alkyl insbesondere Methyl, Ethyl, Propyl, Butyl, Pentyl wobei insbesondere sekundäre und tertiäre Alkylreste genannt seien, $C_{1-6}$-Alkylphenyl das gegebenenfalls durch Halogen, insbesondere Fluor, Chlor, $C_{1-6}$-Alkyl, OH, $C_{1-4}$-Alkoxy, gegebenenfalls halogensubstituiertes Methylendioxy oder Ethylendioxy substituiert sein kann, Phenyl das gegebenenfalls durch Halogen, insbesondere Chlor oder Fluor, $C_{1-4}$-Alkyl, OH, $C_{1-4}$-Alkoxy, gegebenenfalls halogensubstituiertes Methylendioxy oder Ethylendioxy substituiert sein kann, steht,
$R^9$ für Acetyl oder Wasserstoff,
Hal für Chlor steht.

Im einzelnen seien folgende Verbindungen der Formel I genannt:

$R^1 = NHR^8$; $R^2 + R^3 = O$; $R^4$, $R^5 = H$; Hal = Cl,

| $R^8$ | $R^9$ |
|---|---|
| tert.-Butyl | H |
| tert.-Butyl | $\overset{O}{\overset{\|\|}{C}}-CH_3$ |
| i-Propyl | H |
| i-Propyl | $\overset{O}{\overset{\|\|}{C}}-CH_3$ |
| Cyclohexyl | H |

| | |
|---|---|
| Cyclohexyl | $\overset{\overset{O}{\|}}{C}-CH_3$ |
| 2-(1-Phenyl)-propyl | H |
| 2-(1-Phenyl)-propyl | $\overset{\overset{O}{\|}}{C}-CH_3$ |
| Methyl | H |
| Methyl | $\overset{\overset{O}{\|}}{C}-CH_3$ |
| i-Butyl | H |
| i-Butyl | $\overset{\overset{O}{\|}}{C}-CH_3$ |
| sec.-Butyl | H |
| sec.-Butyl | $\overset{\overset{O}{\|}}{C}-CH_3$ |

$R^1$ = $OR^7$; $R^2$ = $R^3$ = O; $R^4$, $R^5$ = H;

| $R^7$ | $R^9$ |
|---|---|
| $-CH_3$ | H |
| $-CH_3$ | $\overset{\overset{O}{\|}}{C}-CH_3$ |
| -Ethyl | H |
| -Ethyl | $\overset{\overset{O}{\|}}{C}-CH_3$ |
| -i-Propyl | H |
| -i-Propyl | $\overset{\overset{O}{\|}}{C}-CH_3$ |

$R^1 = NHR^8;\ R^2, R^3 = H;\ R^4, R^5 = H;\ R^9 = H$

| $R^8$ |
| --- |
| tert.-Butyl |
| i-Propyl |
| Cyclohexyl |
| 2-(1-Phenyl)-propyl |
| Methyl |
| i-Butyl |
| sec.-Butyl |

Setzt man zur Durchführung des erfindungsgemäßen Verfahrens als Verbindung der Formel II 2-(5-Aminopyridyl-2)-2-acetoxyessigsäure-cyclohexylamid und als Halogenwasserstoffsäure HBr ein, so läßt sich der Reaktionsverlauf durch folgendes Formelschema wiedergeben:

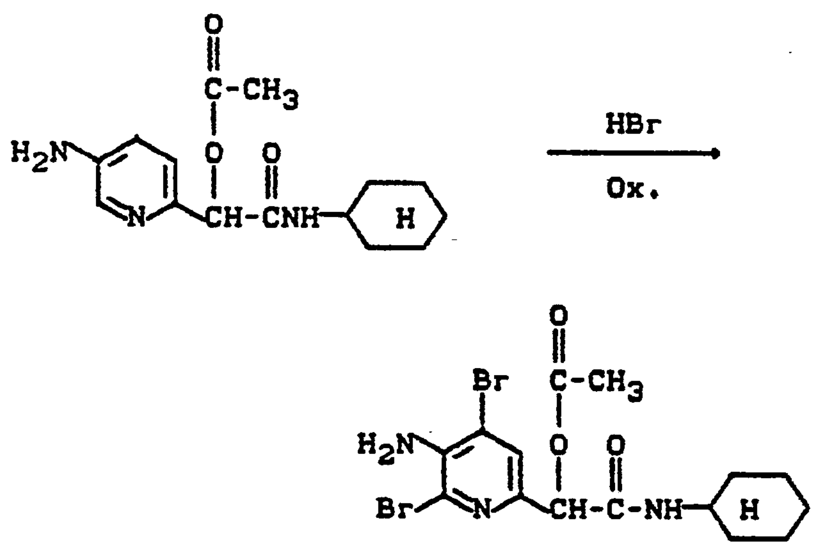


Bevorzugt werden Verbindungen der Formel II eingesetzt, bei denen die Substituenten $R^1$-$R^9$ und Hal die bei den Verbindungen der Formel I angegebenen bevorzugten und besonders bevorzugten Bedeutungen besitzen und $R^9$ für $C_{1-4}$-Alkylcarbonyl insbesondere für Acetyl steht.

Bevorzugt werden zur Halogenierung die Halogenwasserstoffsäuren HCl und HBr, besonders bevorzugt HCl eingesetzt.

Bevorzugt werden als Oxidationsmittel genannt Wasserstoffperoxid, dessen Salze und Derivate wie z.B. Natriumperoxid, Persäuren wie z.B. aliphatische und aromatische Persäuren. Als solche seien genannt Perpropionsäure, Perbenzoesäure, die gegebenenfalls substituiert sein können;

Salze oder Ester der Hypochlorigen Säure wie z.B. Natriumhypochlorit, sowie Kaliumpermanganat, Mangandioxid, Peroxomono- und Peroxodisulfate.

Die Halogenwasserstoffsäure wird bevorzugt als wäßrige konzentrierte Säure, besonders bevorzugt als 20 %ige Säure eingesetzt.

Für den Fall, daß selektiv in 6-Stellung des Pyridinringes monosubstituiert werden soll, wird das Oxidationsmittel in einer Menge von etwa einem Äquivalent eingesetzt.

Für den Fall, daß selektiv in 4 und 6 Stellung des Pyridinringes disubstituiert werden soll, wird das Oxidationsmittel in einer Menge von mindestens 2 Äquivalenten eingesetzt.

Das Verfahren wird bevorzugt in wäßriger Lösung durchgeführt. Es kann auch in organischen Lösungsmitteln, die gegenüber den Reaktionsbedingungen inert sind, durchgeführt werden.

Das Verfahren wird bei Temperaturen zwischen 0 und 150°C, bevorzugt zwischen 10 und 100°C, besonders bevorzugt bei 20-80°C durchgeführt.

Es wird bei Normaldruck gearbeitet.

Wie bereits erwähnt, sind die 5-Aminopyridine der Formel III neu.

Bevorzugt seien 5-Aminopyridine der Formel III genannt in der die Substituenten $R^1$, $R^2$, $R^3$ die bei den Verbindungen der Formel I genannten bevorzugten und besonders bevorzugten Bedeutungen besitzen und $R^9$ für $C_{1-4}$-Alkylcarbonyl insbesondere Acetyl steht.

Im einzelnen seien folgende 5-Aminopyridine der Formel III genannt:

$R^9 = COCH_3$;

| $R^2$ | $R^3$ | $R^1$ |
|---|---|---|
| = O | | $OCH_3$ |
| = O | | $NHC_4H_9$-t |
| = O | | NH-i-Propyl |
| = O | | NH-Cyclohexyl |
| = O | | NH-2-(1-Phenyl)-propyl |
| = O | | NH-Methyl |
| = O | | NH-i-Butyl |
| = O | | NH-s-Butyl |
| H | H | $NHC_4H_9$-t |
| H | H | NH-i-Propyl |
| H | H | NH-i-Butyl |
| H | H | NH-s-Butyl |
| H | H | NH-Cyclohexyl |
| H | H | NH-2-(1-Phenyl)-propyl |

Setzt man zur Herstellung der 5-Aminopyridine der Formel III als Ausgangsverbindung der Formel IV 2-Acetoxy-2-(5-nitropyridyl-2)essigsäureamid ein, läßt sich das Verfahren durch folgendes Formelschema wiedergeben:

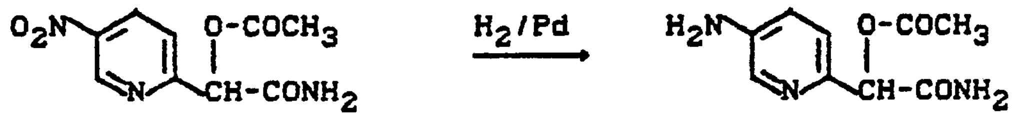


Es werden bevorzugt Verbindungen der Formel IV eingesetzt in welcher $R^1$, $R^2$, $R^3$ und $R^9$ die bei den Verbindungen der Formel III angegebenen bevorzugten und besonders bevorzugten Bedeutungen haben.

Die Reduktion erfolgt katalytisch mit Wasserstoff in Wasser oder organischen Lösungsmitteln oder Lösungsmittelgemischen.

Als Katalysatoren seien z.B. genannt Raney-Nickel, Palladium/Aktivkohle.

Als Lösungsmittel seien z.B. Wasser, Ether wie z.B. Dioxan, aliphatische Säuren wie z.B. Essigsäure, Lösungsmittelgemische wie z.B. Dioxan/Wasser genannt.

Die Reduktion erfolgt bei Temperaturen von 0-100°C bevorzugt bei Raumtemperatur.

Die Reduktion erfolgt bei leichtem Wasserstoffüberdruck von ca. 0,1-1 bar.

Verbindungen der Formel IV sind an sich bekannt. Sie können z.B. nach folgendem Formelschema erhalten werden.

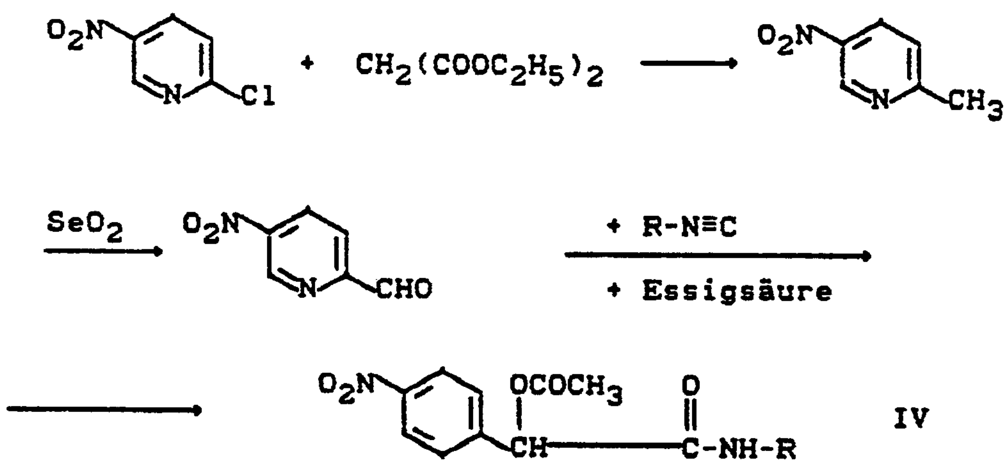


IV

Die Umsetzung von 2-Chlor-5-nitropyridin mit Malonsäureestern ist bekannt (G.H. Cooper et. al. J. Chem. Soc. (C) 1971, S. 772-776). Es wird dabei jedoch in wasserfreiem Medium in Gegenwart von Natriumamid gearbeitet. Die Reaktion läßt sich aber auch wesentlich einfacher und preisgünstiger in Gegenwart von Alkalihydroxiden z.B. NaOH, KOH in inerten polaren aprotischen Lösungsmitteln durchführen. Als Lösungsmittel seien genannt Ether wie Diethyl-, Dibutylether, Glycoldimethylether, Diglycoldimethylether, Tetrahydrofuran, Dioxan, Nitrile wie Acetonitril, Propionitril, Benzonitril, Glutarsäuredinitril, Dimethylacetamid, N-Methylpyrrolidon, DMSO, Tetramethylensulfon, Hexamethylphosphorsäuretriamid, t-Butanol.

Die Reaktion kann auch in Gegenwart von Verbindungen durchgeführt werden, die als Phasentransferkatalysatoren in zweiphasigen Lösungsmittelsystemen dienen.

Als solche seien genannt:

Tetraalkyl- und Trialkylaralkyl-ammoniumsalze mit vorzugsweise 1 bis 10, insbesondere 1 bis 8 Kohlenstoffatomen je Alkylgruppe, vorzugsweise Phenyl als Arylbestandteil der Aralkylgruppe und vorzugsweise Phenyl als Arylbestandteil der Aralkylgruppe und vorzugsweise 1 bis 4, insbesondere 1 oder 2 Kohlenstoffatomen im Alkylteil der Aralkylgruppen. Hierbei kommen vor allem die Halogenide, wie Chloride, Bromide und Iodide, vorzugsweise die Chloride und Bromide in Frage. Beispielhaft seien Tetrabutylammoniumbromid, Benzyl-triethylammoniumchlorid und Methyltrioctylammoniumchlorid genannt.

Zusätzlich zu den oben genannten Verdünnungsmitteln können dann die folgenden Verdünnungsmittel verwendet werden: aliphatische, aromatische, gegebenenfalls halogenierte Kohlenwasserstoffe wie Pentan, Hexan, Heptan, Cyclohexan, Petrolether, Benzin, Ligroin, Benzol, Toluol, Methylenchlorid, Ethylenchlorid, Chloroform, Chlorbenzole.

Die Reaktion wird bei -20 bis 150°C, bevorzugt bei 10 bis 50°C durchgeführt. Nach Beendigung der

Reaktion wird das Gemisch mit Säure neutralisiert, das Lösungsmittel abdestilliert oder das Gemisch auf Wasser gegeben. Das Gemisch kann aber auch ohne Isolierung mit wäßriger Säure wie z.B. HCl gekocht werden und danach das entstandene 5-Nitropicolin z.B. mit Dichlormethan extrahiert werden.

Ein Teil der Verbindungen der Formel I besitzen leitungsfördernde Eigenschaften bei Tieren. Sie bewirken z.B. eine Verschiebung des Fleisch-Fett-Verhältnisses zugunsten von Fleisch bei der Aufzucht von Tieren. Andere Verbindungen der Formel I lassen sich mit Hilfe einfacher Verfahren in solche leistungsfördernde Wirkstoffe überführen. Dann wird auf die ältere, nicht vorveröffentlichte Anmeldung der Anmelderin EP-A 0 244 728 verwiesen.

1. Verfahren zur Herstellung von 5-Amino-4,6-dihalogenpyridinen

Beispiel 1

2-(3-Amino-2-chlor-6-pyridyl)-2-hydroxyessigsäure-tert.-butylamid

12 g (45,3 mmol) 2-(5-Amino-2-pyridyl)-2-acetyloxyessigsäure-tert.-butylamid werden in 90 ml konzentrierter HCl gelöst, auf 45°C vorgeheizt und bei dieser Temperatur innerhalb von 20 Minuten 5,13 ml (45,3 mmol) 30 %ige $H_2O_2$ Lösung zugetropft.

Es wird noch 10 Minuten nachgerührt, auf 750 ml Wasser gegossen und mit 45 %igen NaOH neutralisiert. Das sich abscheidende Öl wird in $CHCl_3$ aufgenommen, die wäßrige Phase nochmals mit $CHCl_3$ nachextrahiert. Die vereinigten Extrakte werden mit $NaHCO_3$-Lösung gewaschen, mit $Na_2SO_4$ getrocknet und im Vakuum eingedampft. Der Rückstand wird am Kieselgel mit $CHCl_3$/Essigester chromatographiert.

Ausbeute: 5,8 g (50 % der Theorie), Schmelzpunkt: 136°C.

Beispiel 2

2-(3-Amino-2,4-dichlor-6-pyridyl)-2-hydroxyessigsäure-tert.-butylamid

10 g (37,7 mmol) 2-Acetoxy-(5-amino-2-pyridyl)-N-tert.-butyl-acetamid werden in 75 ml konz. HCl eingetragen und 10 ml (88,2 mmol) 30 %iges $H_2O_2$ zugetropft. Die exotherme Reaktion wird durch Kühlung mit Eiswasser auf 50-55°C gehalten.

Nach Beendigung des Zutropfens wird noch 10 Minuten nachgerührt, dann auf 375 ml Wasser gegossen und dreimal mit je 200 ml $CHCl_3$ extrahiert. Der Extrakt wird mit $NaHCO_3$-Lösung gewaschen, getrocknet und eingedampft. Der Rückstand wird aus $CHCl_3$/Petrolether umkristallisiert.

Ausbeute: 3,2 g (29 %) Schmelzpunkt: 177°C

Beispiel 3

N-tert.-Butyl-1-(3-amino-2,4-dichlor-6-pyridyl)-ethanolamin

500 mg (2,4 mmol) N-tert.-Butyl-1-(5-amino-2-pyridyl)-ethanolamin werden in 5 ml konzentrierter HCl gelöst und langsam innerhalb von 10 Minuten 0,6 ml (5,3 mmol) 30 %iges $H_2O_2$ zugetropft. Dabei wird die Temperatur durch Kühlung auf 30°C gehalten. Es wird noch 15 Minuten nachgerührt, dann auf 50 ml Wasser gegossen und mit 6 ml 45 %igem NaOH alkalisch gestellt.

Es wird dreimal mit $CHCl_3$ extrahiert, die Extrakte mit $Na_2SO_4$ getrocknet und eingedampft. Rückstand: 310 mg schwachbraunes Öl. Gehalt nach HPLC: 55 %

2. Verfahren zur Herstellung von 5-Aminopyridinen

Beispiel 4

2-(5-Amino-2-pyridyl)-2-acetyloxy-essigsäure-tert.-butylamid

5 g (16,9 mmol) 2-Acetoxy-2-(5-nitro-2-pyridyl)-N-tert.-butyl-acetamid werden in 50 ml 90 %igem wäßrigen Dioxan gelöst, 250 mg 10 % Palladium auf Aktivkohle zugegeben und bei Raumtemperatur bis zur vollständigen Wasserstoffaufnahme hydriert. Zur Aufarbeitung wird der Katalysator abfiltriert und das Filtrat eingedampft. Der Rückstand wird getrocknet.

Ausbeute: 4,4 g (98 %)
Schmelzpunkt: 180°C

analog wurde erhalten:

Beispiel 5

**Beispiel 5**

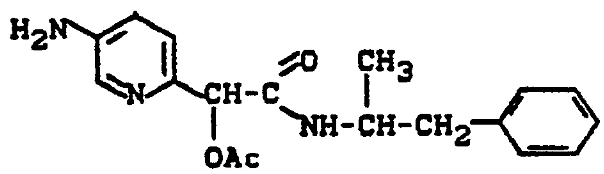

**Schmelzpunkt: 161°C (Diastereomerengemisch)**

Schmelzpunkt: 161°C (Diastereomerengemisch)

3. Verfahren zur Herstellung der 5-Nitro-2-malonylpyridine

Beispiel 6

5-Nitro-2-pyridyl-malonsäurediethylester

Zu 10,8 g (675 mmol) Malonsäurediethylester in 30 ml DMF werden unter Kühlung bei Raumtemperatur 2,7 g (67,5 mmol) gepulverte NaOH eingetragen, anschließend eine Lösung von 5 g (31 mmol) 2-Chlor-5-nitropyridin in DMF zugetropft und bis zur vollständigen Umsetzung bei Raumtemperatur gerührt. Zur Aufarbeitung wird auf 500 ml Wasser gegossen, mit konz. HCl angesäuert, der Niederschlag abgesaugt und mit Petrolether nachgewaschen.

Ausbeute: 192 g (Gehalt 90 %) ≙ 97 % der Theorie.
Analog werden in anderen Lösungsmitteln die folgenden Ausbeuten erhalten:

| Lösungsmittel | Ausbeute (%) |
|---|---|
| Dimethylsulfoxid | 95 |
| N-Methylpyrrolidon | 97 |
| Tetrahydrofuran | 93 |
| Dioxan | 74 |

Beispiel 7

Zu einer Lösung von 25,6 ml (0,15 mol) Methylmalonsäurediethylester in 100 ml DMF werden unter Kühlung 6 g (0,15 mol) frisch gepulverte NaOH eingetragen und anschließend 15,8 g (0,1 mol) 2-Chlor-5-nitropyridin, gelöst in 50 ml DMF, unter Kühlung bei Raumtemperatur zugetropft. Es wird 5 Stunden nachgerührt, danach mit verdünnter Salzsäure neutralisiert und auf 1 l Wasser gegossen. Das sich abscheidende Öl wird in 300 ml Dichlormethan aufgenommen, noch zweimal mit Wasser gewaschen, mit $Na_2SO_4$ getrocknet und im Vakuum eingedampft.
Rückstand: 30,2 g; Gehalt: 88% (HPLC) Ausbeute: 89,8 % der Theorie:

Beispiel 8

2,6 ml (15 mmol) Methylmalonsäurediethylester werden in 50 ml Dichlormethan gelöst, 1,59 g (10 mmol) 2-Chlor-5-nitropyridin, 0,6 g (15 mmol) Tetrabutylammoniumchlorid zugegeben und bei Raumtemperatur 4 Stunden gerührt. Zur Aufarbeitung werden 100 ml Wasser zugegeben und mit Salzsäure neutralisiert. Die organische Phase wird abgetrennt und noch zweimal mit Wasser gewaschen. Nach Abdampfen des Lösungsmittels verbleiben 3,12 g mit einem Gehalt von 86% an Produkt (92,3 % der Theorie).

4. Verfahren zur Herstellung der 5-Nitro-2-alkylpyridine

Beispiel 9

5-Nitro-2-picolin

206 g (1,29 mol) Diethylmalonat werden zu 600 ml DMF gegeben und unter Kühlung 51,2 g (1,28 Mol) frisch gepulverte NaOH eingetragen. Es wird noch 15 Minuten nachgerührt, dann bei Raumtemperatur eine Lösung von 94,8 g (0,6 mol) 5-Nitro-2-chlorpyridin in 600 ml DMF zugetropft. Die entstandene tiefrote Lösung wird bis zur vollständigen Umsetzung gerührt, dann bis zum Farbumschlag nach orange 36 %ige HCl zugetropft und im Vakuum bis zur Trockne eingedampft. Der Rückstand wird in 400 ml $CHCl_3$ aufgenommen, mit 500 ml Wasser gewaschen und nochmals eingedampft. Der Rückstand wird nun in einer Mischung aus 1200 ml 36 %iger HCl und 870 ml Wasser gelöst und bis zur Beendigung der Gasentwicklung unter Rückfluß gekocht. Danach wird im Vakuum eingedampft, der Rückstand in 500 ml Wasser gelöst, mit Dichlormethan extrahiert und die organische Phase nochmals mit Wasser gewaschen. Nach Abzug des Lösungsmittels verbleiben 72 g (87 %) 5-Nitro-2-picolin.
Schmelzpunkt: 109-111°C.

**Patentansprüche**

1. Verfahren zur Herstellung von 5-Amino-4,6-dihalogen-pyridinderivaten der Formel

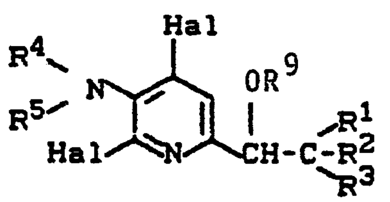

I

in welcher
$R^1$ für Wasserstoff, -O-$R^7$ oder -NH$R^8$ steht,
$R^2$ für Wasserstoff, Halogen steht
$R^3$ für Wasserstoff, oder gemeinsam mit $R^2$ für =O steht,
$R^4$ für Wasserstoff oder Alkyl steht,
$R^5$ für Wasserstoff, Alkyl, Halogenalkyl, Aryl steht,
$R^7$ für Alkyl,
$R^8$ für Alkyl, Cycloalkyl, Aralkyl, Aryl, Heterocyclyl steht, die gegebenenfalls substiutiert sein können,
$R^9$ für Acyl oder Wasserstoff,
Hal für Halogen steht,
dadurch gekennzeichnet, daß man
Verbindungen der Formel II

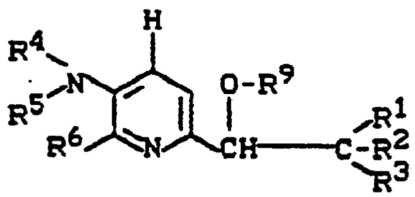

II

in welcher
$R^1$-$R^5$ die oben angegeben Bedeutung besitzen,
$R^6$ für Wasserstoff oder Halogen steht,
$R^9$ für Acyl oder Wasserstoff steht,
mit Halogenwasserstoffsäuren in Gegenwart von Oxidationsmitteln halogeniert.

2. 5-Aminopyridine der Formel III

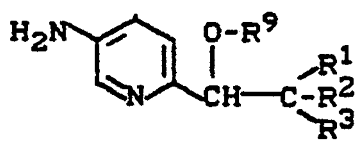

III

in welcher
$R^1$ für Wasserstoff, -O-$R^7$ oder -NH$R^8$ steht,
$R^2$ für Wasserstoff oder Halogen steht,
$R^3$ für Wasserstoff oder gemeinsam mit $R^2$ für =O steht,
$R^9$ für Acyl oder Wasserstoff steht,
$R^7$-$R^8$ die in Anspruch 1 genannten Bedeutungen besitzen.

3. Verfahren zur Herstellung von 5-Aminopyridinen der Formel III

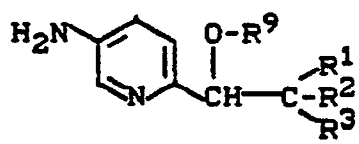

III

in welcher
$R^1$, $R^2$, $R^3$, $R^9$ die in Anspruch 2 genannten Bedeutungen besitzen,
dadurch gekennzeichnet, daß man
5-Nitropyridine der Formel IV

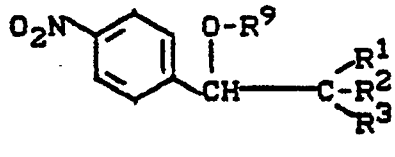

IV

in welcher
$R^1$, $R^2$, $R^3$, $R^9$ die weiter oben genannten Bedeutungen besitzen,
mit Wasserstoff katalytisch reduziert.

**Revendications**

1. Procédé de production de dérivés de 5-amino-4.6-dihalogénopyridines de formule

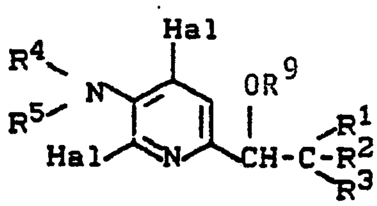

I

dans laquelle
$R^1$ est l'hydrogène, un groupe -O-$R^7$ ou -NH$R^8$,
$R^2$ est l'hydrogène, un halogène,
$R^3$ est l'hydrogène ou représente = 0 conjointement avec $R^2$,
$R^4$ est l'hydrogène ou un groupe alkyle,
$R^5$ est l'hydrogène, un groupe alkyle, halogénalkyle, aryle,
$R^7$ est un groupe alkyle,
$R^8$ est un groupe alkyle, cycloalkyle, aralkyle, aryle, hétérocyclyle, ces groupes pouvant éventuellement être substitués,
$R^9$ est un groupe acyle ou l'hydrogène,
Hal désigne un halogène,

caractérisé en ce qu'on halogène avec des acides halogénhydriques en présence d'agents oxydants, des composés de formule II

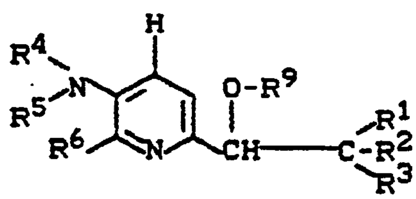

II

dans laquelle
$R^1$–$R^5$ ont la définition indiquée ci-dessus,
$R^6$ est l'hydrogène ou un halogène,
$R^9$ est un groupe acyle ou l'hydrogène.

2.5-aminopyridines de formule III

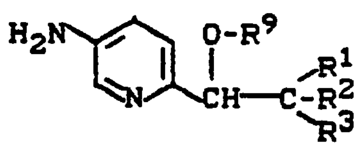

III

dans laquelle
$R^1$ est l'hydrogène, un groupe -O-$R^7$ ou -NH$R^8$,
$R^2$ est l'hydrogène ou un halogène,
$R^3$ est l'hydrogène ou représente =0 conjointement avec $R^2$,
$R^9$ est un groupe acyle ou l'hydrogène,
$R^7$–$R^8$ ont les définitions mentionnées dans la revendication 1.

3. Procédé de production de 5-aminopyridines de formule III

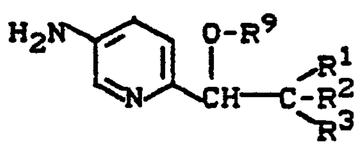

III

dans laquelle
$R^1$, $R^2$, $R^3$, $R^9$ ont les définitions mentionnées dans la revendication 2, caractérisé en ce qu'on réduit par voie catalytique avec de l'hydrogène des 5-nitropyridines de formule IV

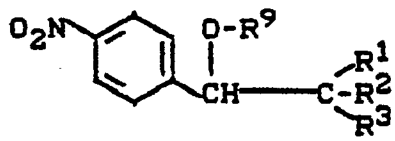

IV

dans laquelle $R^1$, $R^2$, $R^3$, $R^9$ ont les définitions données plus haut.

**Claims**

1. Process for the preparation of 5-amino-4,6-dihalogeno-pyridine derivatives of the formula

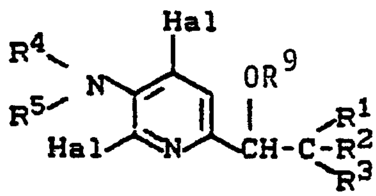

I

in which
$R^1$ represents hydrogen, $-O-R^7$ or $-NHR^8$,
$R^2$ represents hydrogen or halogen,
$R^3$ represents hydrogen or, together with $R^2$, represents =0,
$R^4$ represents hydrogen or alkyl,
$R^5$ represents hydrogen, alkyl, halogenoalkyl or aryl,
$R^7$ represents alkyl,
$R^8$ represents alkyl, cycloalkyl, aralkyl, aryl or heterocyclyl, which can optionally be substituted,
$R^9$ represents acyl or hydrogen and Hal represents halogen, characterized in that compounds of the formula II

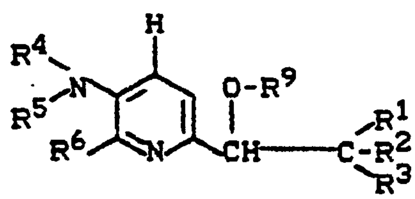

II

in which
$R^1$-$R^5$ have the abovementioned meaning,
$R^6$ represents hydrogen or halogen and
$R^9$ represents acyl or hydrogen, are halogenated with hydrogen halide acids in the presence of oxidizing agents.

2. 5-Aminopyridines of the formula III

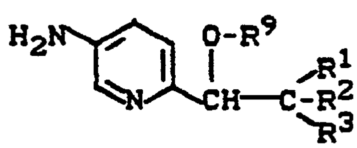

III

in which
$R^1$ represents hydrogen, $-O-R^7$ or $-NHR^8$,
$R^2$ represents hydrogen or halogen,
$R^3$ represents hydrogen or, together with $R^2$, represents =0
$R^9$ represents acyl or hydrogen and
$R^7$-$R^8$ have the meanings given in Claim 1.

3. Process for the preparation of 5-aminopyridines of the formula III

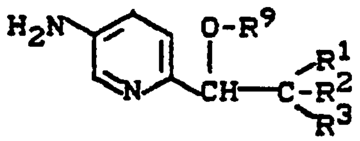

III

in which

$R^1$, $R^2$, $R^3$ and $R^9$ have the meanings given in Claim 2, characterized in that 5-nitropyridines of the formula IV

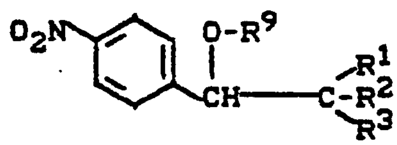

IV

$R^1$, $R^2$, $R^3$ and $R^9$ have the meanings given above, are reduced catalytically with hydrogen.